(19) **Europäisches Patentamt**

**European Patent Office**

**Office européen des brevets**

(11) Veröffentlichungsnummer: **0 051 686**
**B1**

(12) **EUROPÄISCHE PATENTSCHRIFT**

(45) Veröffentlichungstag der Patentschrift: **30.07.86**

(21) Anmeldenummer: **80106835.4**

(22) Anmeldetag: **06.11.80**

(51) Int. Cl.⁴: **A 61 F 2/30**

(54) Gelenkendoprothese.

(43) Veröffentlichungstag der Anmeldung:
**19.05.82 Patentblatt 82/20**

(45) Bekanntmachung des Hinweises auf die
Patenterteilung:
**30.07.86 Patentblatt 86/31**

(84) Benannte Vertragsstaaten:
**CH DE FR GB IT LI NL SE**

(56) Entgegenhaltungen:
**DE-A-2 039 731**
**DE-A-2 358 159**
**DE-B-2 235 874**
**DE-C- 762 762**
**DE-U-7 739 054**
**FR-A-2 437 199**
**US-A-2 010 367**
**US-A-3 818 512**
**US-A-3 848 272**
**US-A-4 044 403**

(73) Patentinhaber: **Waldemar Link GmbH & Co**
**Barkhausenweg 10**
**D-2000 Hamburg 63 (DE)**

(72) Erfinder: **Keller, Arnold**
**An der Naher Furth 5**
**D-2062 Kaihude (DE)**

(74) Vertreter: **Glawe, Delfs, Moll & Partner**
**Patentanwälte**
**Rothenbaumchaussee 58 Postfach 2570**
**D-2000 Hamburg 13 (DE)**

Courier Press, Leamington Spa, England.

**Beschreibung**

Die Erfindung betrifft eine Kugelgelenk-Endoprothese mit einer einen Gelenkopf umfassenden Pfanne, die aus einem Mantelteil und einem die Pfannenlagerfläche bildenden Einsatz aus Kunststoff besteht, die als Luxationssicherung eine ebenfalls aus Kunststoff bestehende, an den Gelenkkopf heranreichende Öffnungsverengung aufweist, sowie mit einem geteilten Sicherungsring, der in einer an der Pfanne gebildeten Ringnut, diese vollständig füllend, gehalten ist, wobei der die Ringnut auf der Öffnungsseite begrenzende Bund an einer Stelle ausgeschnitten ist zum Einschieben bzw. Ausziehen des geteilten Rings in Umfangsrichtung, der dafür an wenigstens einem Ende eine Handhabe für ein Werkzeug aufweist.

Bei bekannten Hüftgelenk-Endoprothesen (DE—A—23 58 159; FR—A—24 37 199) ist die Öffnung des die Pfannenlagerfläche bildenden Einsatzes aus Kunststoff gegenüber ihrem Größtdurchmesser verengt ausgeführt, und zwar so stark, daß der Prothesenkopf eben noch unter elastischer Verformung in die Pfanne eingesetzt werden kann. Die auf diese Weise erzielbare Luxationssicherung ist jedoch begrenzt, zumal der den Einsatz umfassende Mantelteil die elastische Verformbarkeit des Kunststoffeinsatzes beschränkt. — Eine stärkere Verengung der Öffnung des Kunststoffeinsatzes läßt sich dann erreichen, wenn dieser geteilt ausgeführt wird, wie dies bei der eingangs erwähnten bekannten Prothese der Fall ist (US—A—38 48 272). Dies zwingt zu einer wesentlich sichereren Halterung des Kunststoffeinsatzes im Mantelteil als bei denjenigen Konstruktionen, die sich eines ungeteilten Kunststoffeinsatzes bedienen. Diese Sicherung wird bei der bekannten Prothese dadurch erzielt, daß die Einsatzteile an ihrem Außenumfang und der Mantelteil an seinem Innenumfang einander gegenüberstehende Ringnuten aufweisen, wobei der Bund, der diese Ringnuten auf der Öffnungsseite jeweils begrenzt, an einer Stelle ausgeschnitten ist zum Einschieben bzw. Ausziehen eines Rings, der jeweils zur Hälfte in die eine und in die andere Ringnut eingreift und dadurch den Kunststoffeinsatz formschlüssig mit dem Mantelteil verbindet.

Die geteilte Ausführung des Kunststoffeinsatzes ist nachteilig, weil dadurch Verschleißschäden gefördert werden. Auch ist die Konstruktion insgesamt kompliziert.

Der Erfindung liegt daher die Aufgabe zugrunde, eine Prothese der eingangs genannten Art zu schaffen, die ein gute Luxationssicherung mit der Verwendbarkeit eines ungeteilten Kunststoffeinsatzes verbindet.

Die erfindungsgemäße Lösung besteht darin, daß die Öffnungsverengung von dem aus Kunststoff bestehenden Sicherungsring gebildet ist, der mit mehr als seiner halben Radialausdehnung in der in dem Kunststoffeinsatz gebildeten Ringnut eingeschlossen ist.

Während bei der bekannten Prothese der Sicherungsring fern von den eigentlichen Lagerflächen lediglich die Konstruktionsteile der Prothese zusammenhält, nimmt er im Falle der Erfindung an der Bildung der Lagerfläche teil, weil er selbst die Öffnungsverengung bildet, die den Gelenkkopf am Entweichen aus der Pfanne hindert. Der Kunststoffeinsatz kann mit anderen, herkömmlichen Mitteln, beispielsweise einer Schnappverbindung, im Mantelteil gehalten sein.

Auf dem Gebiet der Kraftfahrzeugtechnik sind Kugelgelenke bekannt, bei denen ein in einer Nut der Pfanne gelegener Ring den Gelenkkopf in der Pfanne sichert. Dabei kann ein aus Federstahl bestehender Ring in einer Nut derart aufgenommen sein, daß der Kugelkopf durch den nach außen ausweichenden Sicherungsring hindurch in die Pfanne eingedrückt werden kann, nicht aber daraus entweichen kann (US—A—20 10 367). Dabei ist der Sicherungsring im Hinblick auf die vom ihm übertragenden Kräfte auch dann in dem aus Metall bestehenden Teil der Pfanne gehalten, wenn diese zwecks Reibungsverringerung einen Einsatz enthält (DE—B—22 35 874). Bekannt ist auch die Verwendung eines Sicherungsrings aus biegsamen Werkstoff, beispielsweise thermoplastischem Kunststoff oder Weichgummi, der in die in dem metallenen Pfannenteil enthaltene Nut durch eine tangential angeordnete Öffnung eingeschoben wird (DE—C—7 62 762).

Dem Arzt oder Prothesentechniker, den die Entwicklung neuer Prothesen obliegt, ist zwar die Existenz von Kugelgelenken und deren grundsätzlicher Aufbau bekannt; jedoch ist nicht anzunehmen, daß er über spezielle Kenntnisse der Kugelgelenkbauweise auf dem Gebiet der Kraftfahrzeugtechnik verfügt. Man kann daher nicht ohne weiteres voraussetzen, daß Konstruktionsanregungen aus dem Gebiet der Kraftfahrzeugtechnik vom Arzt oder Prothesentechniker leicht übernommen werden können. Darüber hinaus konnten die genannten Schriften für den Arzt oder Prothesentechniker keine unmittelbar verwendbaren Anregungen für die Konstruktion von Gelenkendoprothesen geben, weil die Benutzungsvoraussetzungen und -beanspruchungen sehr unterschiedlich sind. Während es im Bereich der Kraftzeugtechnik möglich ist, ein schadhaftes Teil ohne weiteres gegen ein neues auszutauschen, ist dies im Bereich der Endoprothetik nur unter großer Schwierigkeiten und Leiden des Betroffenen möglich; Endoprothesen müssen daher im Rahmen des Möglichen absolut zuverlässig sein. Alle Störungsquellen, die den Verschleiß verstärken oder die Halterung des Sicherungsrings in der Pfanne gefährden könnten, mußten daher ausscheiden. Nachdem man schon bei Kraftfahrzeug-Kugelgelenken die Halterung des Sicherungsrings im metallenen Pfannen- oder Pfannenmantelteil für notwendig gehalten hatte, hätte man annehmen müssen, daß dies erst recht auf dem Gebiet der Endoprothetik erforderlich sein müßte, um dem Sicherungsring die erforderliche Halterungssicherheit zu geben. Davon abweichend hat die Erfindung die Haltenut für den Sicherungsring jedoch im Kunststoffeinsatz angeordnet, obwohl dieser prinzipiell eine wesentlich geringere Festigkeit als der metallene Mantelteil zu bieten hat. Eine Unterbringung im Mantelteil kam nicht in Frage, weil der Kunststoffeinsatz einer Endoprothese verhältnismäßig dickwandig sein muß, um hinreichende Verschleißreserve zu bieten, und die Überbrückung der Radiusdistanz zwischen dem metallenen Mantelteil und dem Gelenkkopf nur durch einen metallenen Sicherungsring zu überwinden gewesen wäre, der aber deshalb nicht in Frage kam, weil er die empfindliche Gelenkkopfoberfläche durch Verkratzen gefährdet haben würde. Für den Kontakt mit der Gelenkkopfoberfläche kommen im Fall von Endoprothesen vielmehr ausschließlich Kunststoffteile in Betracht, deren Festigkeit für die Überbrückung der Distanz zwischen dem Mantelteil der Pfanne und dem Gelenkkopf nicht ausreicht. — Die Erfindung nutzt nun die größere Dicke des Kunststoff-Protheseneinsatzes dadurch, daß sie darin eine Haltenut für den Sicherungsring unterbringt, dessen Tiefe größer ist als die halbe Radialausdehnung des Sicherungsrings, so daß dieser mit einer die Schwäche des Materials kompensierenden größeren Zuverlässigkeit darin gehalten ist.

Dieser Ring kann ohne Rücksicht auf den maximalen Prothesenkopfdurchmesser so bemessen werden, daß eine Luxation im allgemeinen ausgeschlossen ist.

Zweckmäßigerweise ist die wenigstens an einem Ende des Rings vorgesehene Handhabe für ein Auszeihwerkzeug als Bohrung ausgebildet. Ferner hat es sich als zweckmäßig erwiesen, wenn der Ring zumindest auf seiner außen zu liegen bestimmten Seite geriffelt ist, damit er in Umfangsrichtung verschoben werden kann, wenn er herausgenommen werden soll und sein Ende sich nicht bei dem Ausschnitt befindet.

Obwohl der erfindungsgemäße Ring eine ausreichende Luxationssicherung bildet, kann zusätzlich noch die eingangs erwähnte, an sich bekannte Pfannenverengung im Öffnungsbereich vorgesehen sein. Dies entlastet einerseits den Ring und führt andererseits zu einer sichereren Halterung des Rings, da dieser in einem geringeren Pfannendurchmesser gehalten werden kann, als wenn die erwähnte Verengung nicht vorhanden wäre.

Die Erfindung ist nicht auf die Anwendung bei Hüftgelenkprothesen beschränkt. Vielmehr kann ihr Prinzip auch bei anderen Kugelgelenken oder kugelähnlichen Gelenken angewendet werden, beispielsweise am Schultergelenk, Handgelenk, an Finger- und Zehengelenken.

Die Erfindung wird im folgenden näher unter Bezugnahme auf die Zeichnung erläutert. Darin zeigen:

Fig. 1 einen Längsschnitt und

Fig. 2 eine Draufsicht auf die Öffnungsseite der erfindungsgemäßen Pfanne.

Die Pfanne besteht aus einem Metallmantel 1 und einem Kunststoffeinsatz 2, der vorzugsweise aus hoch molekularem Niederdruck-Polyethylen besteht, der mit einer Ringnase 3 in einer Ringnut 4 des Metallmantels gesichert ist. Die Pfanne umfaßt den strichpunktiert angedeuteten Prothesenkopf 5 zu mehr als 90°, nämlich bis zum Durchmesser 6, der etwas kleiner ist als der Maximaldurchmesser bei 7. Insoweit kann die Anordnung als bekannt betrachtet werden.

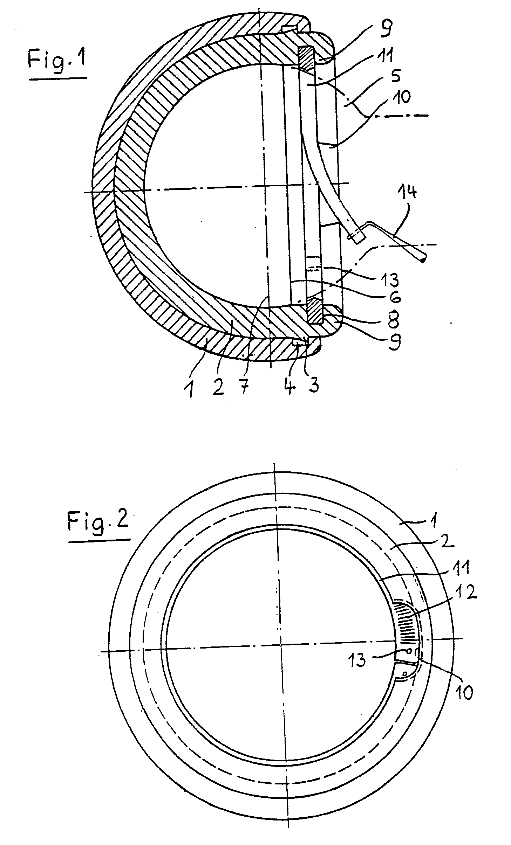

Öffnungsseitig des Durchmessers 6 ist die Innenfläche der Pfanne im wesentlichen zylindrisch ausgebildet mit einer eingestochenen Ringnut 8, die von zwei parallelen Ebenen senkrecht zur Rotationsachse begrenzt ist. Der die Nut 8 öffnungsseitig begrenzende Bund 9 besitzt einen Ausschnitt 10, der sich über die volle radiale Tiefe der Nut erstreckt und in Umfangsrichtung eine Ausdehnung besitzt, die etwa der drei- bis sechsfachen Breite der Nut in Axialrichtung entspricht. In der Nut sitzt der geteilte Ring 11, dessen außenseitige Oberfläche, wie bei 12 gezeigt, geriffelt ist und dessen Teilungsenden kleine Bohrungen 13 in Axialrichtung enthalten, in die die Spitze eines Hakenwerkzeugs 14 eingesetzt werden kann. Im Normalzustand ist der Ring vollständig in der Nut 10 enthalten. Er hat eine so große radiale Ausdehnung nach innen, daß er sich der Oberfläche des Prothesenkopfs 5 bis auf die übliche Toleranz nähert. Er ist auf seiner radial innenliegenden Fläche dachförmig beiderseits abgeschrägt, damit er richtungsunabhängig eingesetzt werden kann, wobei jeweils die eine oder die andere Schrägfläche mit der Kopfoberfläche zusammenwirkt. Der Ring kann, wie in Fig. 1 gezeigt, unter entsprechender Biegung durch den Ausschnitt 10 in die Nut eingeführt oder aus ihr herausgezogen werden. Wenn er herausgezogen werden soll, wird, er zunächst so in Umfangsrichtung mit Hilfe des Werkzeugs an der Riffelung 12 gedreht, daß das herauszuziehende Teilungsende im Ausschnitt 10 liegt. Danach kann dieses Teilungsende durch Eingriff des Werkzeugs 14 in die zugehörige Bohrung aus dem Ausschnitt 10 herausgebogen und herausgezogen werden.

Der Ring 11 ist in der Nut 8 sicher gehalten, da diese im Verhältnis zur radialen Ausdehnung des Rings eine beträchtliche Tiefe besitzt, die zweckmäßigerweise größer ist also die halbe Radialausdehnung des Rings.

Der Ring 11 besteht zweckmäßigerweise aus demselben Material wie der Pfanneneinsatz 2. Sein entspannter Durchmesser kann größer sein als der Durchmesser des Bodens der Nut 8, damit er umso sicherer in der Nut gehalten ist.

**Patentansprüche**

1. Kugelgelenk-Endoprothese mit einer einen Gelenkkopf (5) umfassenden Pfanne, die aus einem Mantelteil (1) und einem die Pfannenlagerfläche bildenden Einsatz (2) aus Kunststoff besteht, die als Luxationssicherung eine ebenfalls aus Kunststoff bestehende, an den Gelenkkopf heranreichende Öffnungsverengung aufweist, sowie. mit einem geteilten Sicherungsring (11), der in einer an der Pfanne gebildeten Ringnut (8), diese vollständig füllend, gehalten ist, wobei der die Ringnut (8) auf der Öffnungsseite begrenzende Bund (9) an einer Stelle ausgeschnitten (10) ist zum Einschieben bzw. Ausziehen des geteilten Rings (11) in Umfangsrichtung, der dafür an wenigstens einem Ende eine Handhabe (13) für ein Werkzeug (14) aufweist, dadurch gekennzeichnet; daß die Öffnungsverengung von dem aus Kunststoff bestehenden Sicherungsring (11) gebildet ist, der mit mehr als seiner halben Radialausdehnung in der in dem Kunststoffeinsatz (2) gebildeten Ringnut (8) eingeschlossen ist.

2. Kugelgelenk-Endoprothese nach Anspruch 1, dadurch gekennzeichnet, daß beide Ringenden als Handhabe für ein Werkzeug eine Bohrung (13) enthalten.

3. Kugelgelenk-Endoprothese nach Anspruch 1 oder 2, dadurch gekennzeichnet, daß der Ring (11) zumindest auf seiner nach außen gewendeten Seite geriffelt (12) ist.

**Revendications**

1. Endoprothèse d'énarthrose, comprenant un cotyle qui entoure une tête articulaire (5) et qui se compose d'une partie d'enveloppe (1) et d'une garniture (2) en matière plastique qui forme la surface portante du cotyle et qui présente, en tant que protection contre la luxation, un resserrement de son ouverture, également fait de matière plastique et s'étendant jusqu'au contact de la tête articulaire, ainsi qu'un anneau d'arrêt fendu (11) qui est retenu dans une gorge annulaire (8) formée sur le cotyle en remplissant entièrement celle-ci, le collet (9) qui délimite la gorge annulaire (8) du côté de l'ouverture étant entaillé en un point (10) en vue de l'insertion ou de l'extraction en direction circonférentielle de l'anneau fendu (11) qui présente à cet effet, au moins à une extrémité, une prise (13) pour un outil (14), caractérisée en ce que le resserrement de l'ouverture est constitué par l'anneau d'arrêt (11) fait de matière plastique, qui est contenu, par plus de sa demi-dimension radiale, dans la gorge annulaire (8) formée dans la garniture de matière plastique (2).

2. Endoprothèse d'énarthrose selon la revendication 1, caractérisée en ce que les deux extrémités de l'anneau comportent une forure (13) destinée à servir de prise pour un outil.

3. Endoprothèse d'énarthrose selon la revendication 1 ou 2, caractérisée en ce que l'anneau (11) est strié (12), au moins sur sa face dirigée vers l'extérieur.

**Claims**

1. A ball-and-socket joint endoprosthesis with a socket which embraces a joint head (5), which socket comprises a casing part (1) and a plastics insert (2) forming the socket bearing surface and which, for the purpose of preventing dislocation, has an opening constriction likewise made of plastics material and extending up to the joint head, and also with a split retaining ring (11) which is held in and completely fills an annular groove (8) formed in the socket, wherein a flange (9) which bounds the annular groove (8) on the opening side is recessed at one place for the insertion or extraction of the split ring (11) in the circumferential direction, which ring has for this purpose at at least one end a grip (13) for an implement (14), characterised in that the opening constriction is formed by the retaining ring (11) made of plastics material, which is inserted in the annular groove (8) formed in the plastics insert (2) by more than half its radial extent.

2. A ball-and-socket joint endoprosthesis according to Claim 1, characterised in that both ring ends include a bore (13) to provide purchase for an implement.

3. A ball-and-socket joint endoprosthesis according to Claim 1 or 2, characterised in that the ring (11) is grooved (12) at least on its outwardly facing side.

0 051 686

Fig.1

Fig.2

1